Europäisches Patentamt

European Patent Office

Office européen des brevets

(11) Veröffentlichungsnummer: **0 263 371**
**A2**

(19)

(12) **EUROPÄISCHE PATENTANMELDUNG**

(21) Anmeldenummer: 87114015.8

(22) Anmeldetag: 25.09.87

(51) Int. Cl.⁴ **C12M 3/00**

(30) Priorität: 04.10.86 DE 3633891

(43) Veröffentlichungstag der Anmeldung:
**13.04.88 Patentblatt 88/15**

(84) Benannte Vertragsstaaten:
**AT BE CH DE ES FR GB GR IT LI LU NL SE**

(71) Anmelder: **Akzo N.V.**
**Postbus 186 Velperweg 76**
**NL-6800 LS Arnhem(NL)**

(72) Erfinder: **Mark, Ute, Dipl.-Biol.**
**Graaf-Lodewijk-Straat 76**
**NL-6821 Arnhem(NL)**
Erfinder: **Tretzel, Joachim, Dr. Dipl.-Chem.**
**Bessenbacher Weg 45**
**D-8750 Aschaffenburg(DE)**

(74) Vertreter: **Fett, Günter**
**Akzo GmbH Kasinostrasse 19-23**
**D-5600 Wuppertal 1(DE)**

(54) **Verfahren und Vorrichtung zum Kultivieren von tierischen Zellen.**

(57) Verfahren und Vorrichtung zum Kultivieren tierischer Zellen zur Vermehrung derselben und/oder Gewinnung von gewünschten Wertstoffen aus den Zellen, wobei sich in einem Kapillarmembran-Modul Zellen im extrakapillären Raum in einem wäßrigen Kulturmedium befinden und die Versorgung mit Nährstoffen und die Entsorgung von Metaboliten durch das Innere der Kapillarmembranen erfolgt. Im extrakapillären Raum ist ein Trägermaterial für die Zellen angeordnet, auf dem die Zellen bevorzugt adhärieren. Dadurch werden die Membranoberflächen freigehalten, eine gleichbleibend optimale Ver-und Entsorgung der Zellen ist somit gewährleistet. In einer bevorzugten Ausführungsform stellen die Kapillarmembranen die Schußfäden und das Trägermaterial für die Zellen die Kettfäden einer gewebten Matte dar, die beispielsweise in aufgerollter Form in ein Modulgehäuse eingebracht ist.

EP 0 263 371 A2

## Verfahren und Vorrichtung zum Kultivieren von tierischen Zellen

Die Erfindung betrifft ein Verfahren zum Kultivieren von tierischen Zellen zur Vermehrung derselben und/oder Gewinnung von gewünschten Wertstoffen aus den Zellen, wobei sich in einem wenigstens eine - schlauchförmige Membran enthaltenden Mudul Zellen im Raum außerhalb der wenigstens einem - schlauchförmigen Membran in einem wäßrigen Kulturmedium befinden und durch das Innere der wenigstens einen schlauchförmigen Membran die Versorgung der Zellen mit Nährstoffen und die Entsorgung von Metaboliten erfolgt, und eine hierzu geeignete Vorrichtung.

Ein solches Verfahren ist bereits aus der GB-PS 1 395 291 bekannt. Diese beschreibt das Kultivieren von tierischen Zellen auf semipermeablen Kapillarmembranen,durch die die Versorgung der Zellen mit Nährstoffen und Sauerstoff und die Entsorgung von Metaboliten erfolgen.

Bei dem bekannten Verfahren werden die Kapillarmembranen sehr schnell vollständig von den Zellen bedeckt, weil die Zellen an der Membranoberfläche adhärieren und damit eine Überversorgung der unmittelbar an der Oberfläche adhärierenden Zellen und eine Unterversorgung sowie eine schlechte Entsorgung der übrigen im extrakapillären Raum enthaltenen Zellen bedingen, wodurch der zur Zellkultur zur Verfügung stehende extrakapilläre Raum nur ineffektiv genutzt werden kann.

Weitere Verfahren sind beispielsweise in der EP-A 155 237 oder der EP-A 164 813 beschrieben.

Im Rahmen dieser Anmeldung ist der Begriff "Adhäsion von Zellen" so definiert, daß zwischen der Oberfläche von Zellen und der Oberfläche des Trägermaterials,direkt oder indirekt über brückenbildende Substanzen, eine Wechselwirkung stattfindet, die zur Folge hat, daß die Zellen im extrakapillären Raum nicht mehr frei beweglich sind.

Diese Anheftung muß dabei nicht abhängig sein von sichtbaren morphologischen oder physiologischen Veränderungen der Zellen nach der primären Wechselwirkung mit dem Trägermaterial (z.B. wie im Falle anheftungsbedürftiger Zellen durch Abflachung und Zellspreizung).

Als schlauchförmige Membranen werden im Rahmen dieser Anmeldung Membranen mit einem durchgehenden Hohlraum und einer beliebigen (d.h. z.B. runden, ovalen oder auch unsymmetrischen) Querschnittsform bezeichnet.

Kapillarmembranen als spezielle Ausführungsform der schlauchförmigen Membranen weisen im Rahmen dieser Anmeldung einen im wesentlichen kreisförmigen Querschnitt mit einem Außendurchmesser im Bereich von 150 bis 3 500 μm sowie eine im wesentlichen gleichmäßige Wanddicke im Bereich von 5 bis 1 000 μm auf.

Die Aufgabe der vorliegenden Erfindung war es, ein Verfahren zur Verfügung zu stellen, bei dem anheftungsbedürftige und/oder nichtanheftungsbedürftige Zellen unter weitgehender Ausnutzung des zur Verfügung stehenden extrakapillären Modulraumes aus tierischen Zellsuspensionen kultiviert und vermehrt werden können sowie auch gewünschte Wirkstoffe aus den Zellen gewinnbar sind. Gelöst wird die Aufgabe mit einem Verfahren, wie es in den Ansprüchen beschrieben ist.

Es hat sich überraschend herausgestellt, daß bei Verwendung von Geweben, Gewirken o.ä., bei denen die Kapillarmembranen die Schußfäden und textile Fäden die Kettfäden darstellen, die Zellen bevorzugt an den Kettfäden adhärieren, insbesondere in und nach der Inokulationsphase, so daß die Kapillaroberflächen von Zellen weitgehend frei bleiben und für einen ungehinderten Stoffaustausch zur Verfügung stehen. Die Kettfäden fungieren somit als Trägermaterial für die Zellen. Insbesondere gilt dieses, wenn die Abstände der Schußfäden das 0,5-bis 3-fache des Schußfadendurchmessers betragen. Die Abstände der Kettfäden sollen vorzugsweise mindestens das 1,0-fache und höchstens das 100-fache des Kettfadendurchmessers betragen. Das bevorzugte Verhältnis des Durchmessers der Schußfäden zum Durchmesser der Kettfäden liegt im Bereich zwischen 0,4 und 2,0.

Je nach Zelltyp adhärieren die Zellen bevorzugt auf bestimmten Polymeren, wie verseiftem Celluloseacetat, gesättigtem Polyester und/oder Copolyester, Polyurethan, Polyacrylnitril, aromatischem Polyamid, sowie auf Kieselsäure und Kohlenstoff. Eine Anpassung des Trägermaterials für die Zellen an die Adhärenz der Zelltypen läßt sich dadurch erreichen, daß die Fäden des Trägermaterials für die Zellen beschichtet werden. Als Beschichtungsmaterialien kommen beispielsweise Avivagelösungen infrage, vorzugsweise werden die Fäden mit Polyurethan beschichtet.

Die Beschichtung kann auch mit biologischen Substanzen wie z.B. Fibronectin oder Kollagen durchgeführt werden.

Wesentlich für die Auswahl des Membranmaterials ist die Zusammensetzung des Nährmediums und der anfallenden Metabolite. Die Membranen sollen für die Stoffe nur einen geringen diffusiven Widerstand bilden, wohingegen der diffusive Widerstand für die Produkte wie z.B. monoklonale Antikörper oder Metabolite hoch sein soll. Das Nährmedium enthält im allgemeinen Salze, Vitamine, Kohlehydrate und Aminosäuren als niedermolekulare Stoffe, sowie Serum bzw. Serumersatz.

Die gestellten Anforderungen erfüllen insbesondere Membranen aus regenerierter Cellulose, insbesondere solche, die aus Cuoxamlösungen regeneriert wurden. Solche Membranen sind auch dampfsterilisierbar, ohne daß dadurch die Permeabilität für die Nährstoffe und Metabolite beeinrächtigt wird, wenn die Membranen während und nach der Sterilisation feucht gehalten werden. Dampfsterilisation hat gegenüber der üblicherweise für solche Systeme benutzten Ethylenoxidsterilisation den Vorteil, daß die Module nach der Sterilisation nicht entgast werden müssen und keine toxischen Rückstände im Modul zu erwarten sind.

Besonders dann, wenn durch den Anteil höhermolekularer Stoffe im Nährmedium oder bei den Metaboliten eine höhere Permeabilität erforderlich ist, lassen sich auch Membranen aus mikroporösen synthetischen Polymeren mit Vorteil einsetzen. Der maximale mittlere Porendurchmesser bei diesen Membranen beträgt vorzugsweise 0,05 bis 0,2 µm, wobei der mittlere Porendurchmesser mittels der Methode nach MARSHALL, J.C., MELTZER, T.H., veröffentlicht in Bull. Parenteral Drug Assoc., 30, 214 (1976), bestimmt wird. Solche Membranen halten tierische Zellen wie ein Filter zurück. Darüber hinaus weisen sie aber auch eine gute Gasdurchlässigkeit auf, so daß durch solche Membranen auch die Sauerstoffversorgung der Zellen erfolgen kann. Zur verbesserten Sauerstoffversorgung können neben Cellulosekapillarfäden auch gleichzeitig andere Membrankapillarfäden eingesetzt werden, die im wesentlichen dann der Sauerstoffversorgung dienen. Geeignet dazu sind neben mikroporösen synthetischen Membranen auch typische Gasmembranen,wie beispielsweise Silikonkapillarmembranen. In einer besonderen Ausführungsform sind deshalb für die Erfindung Gewebe oder Gewirke bevorzugt, die unterschiedliche Membrankapillarfadentypen in alternierender Anordnung enthalten.

Im allgemeinen werden die Gewebe oder Gewirke aufgewickelt, in ein Modulgehäuse eingebracht und an den Enden mit einem Polyurethan fluiddicht vergossen. Im fertigen Modul liegen dann die Kapillarmembranen im wesentlichen parallel zueinander und zur Gehäuseachse. Die Gewebe oder Gewirke können auch gefaltet in ein Modulgehäuse gebracht werden und dann ebenfalls wiederum mit Polyurethan zur Abdichtung vergossen werden. Eine weitere Möglichkeit besteht darin, daß man ein rechteckiges Modulgehäuse verwendet und in dieses Lagen aus den Geweben oder Gewirken einbringt und entsprechend wiederum an den Stirnseiten beispielsweise mit Polyurethan fluiddicht vergießt. Vorzugsweise werden zwischen die Gewebelagen Abstandshalter eingelegt. Diese können Gewebe, Gewirke, Fadenscharen, Vliese, polyurethanbeschichtete Kügelchen, Fasern oder Schaumstofflagen sein. Durch die Abstandshalter wird der extrakapilläre Raum relativ zum kapillären Raum vergrößert.

Durch das fluiddichte Vergießen sämtlicher Trennfugen am Gehäuse ergibt sich die vorteilhafte Eigenschaft, daß der Modul dampfsterilisierbar ist und bei der Dampfsterilisierung dicht bleibt. Durch die Anwendung eines geeigneten Sterilisationsverfahrens (wie beispielsweise aus der DE-PS 28 11 551 bekannt) läßt sich auf einfache Weise eine Langzeitwirkung der Sterilität verifizieren, die für eine erfolgreiche Zellkultivierung essentiell ist.

Wie bereits vorstehend erläutert, adhärieren tierische Zellen unterschiedlich stark an bestimmten Gebilden, wobei hierfür u.a. die chemische Zusammensetzung und/oder die Struktur des Gebildes maßgeblich ist. Durch Beschichtung läßt sich die Adhärierungsfähigkeit von Zellen beeinflussen. Gebilde, die auf Grund ihrer chemischen Zusammensetzung und/oder ihrer Struktur und/oder ihrer Beschichtung für die Adhärenz von Zellen an diese eine gute Eignung zeigen, stellen das Trägermaterial für die Zellen dar, wobei das Gebilde aus einem Gelege, Gewebe, Gewirke, Vlies, Kügelchen, Fasern,einer Fadenschar oder einem Schaumstoff bestehen kann. Somit ergibt sich eine bevorzugte Ausführungsform der vorliegenden Erfindung daraus, daß die Abstandshalter ebenso das Trägermaterial für die Zellen darstellen können. Damit ist auch ein definierter Abstand vorgegeben, durch den aufgrund der verbesserten Versorgung die Zellen bevorzugt adhärieren und wachsen.

Für das wirkungsvolle Kultivieren von tierischen Zellen zum Zwecke der Vermehrung und/oder Gewinnung von gewünschten Wertstoffen aus den Zellen ist eine gute Versorgung mit Nährstoffen erforderlich. Um dieses sicherzustellen, wird beim erfindungsgemäßen Verfahren vorzugsweise die Versorgung und/oder Entsorgung mittels einer Steuer-Kontroll-Einheit überwacht.

Zur kontaminationssicheren Probenentnahme wird eine Vorrichtung in Form eines Dreiwegeventils verwendet, das zwischen zwei aufeinanderfolgenden Probenentnahmen mit einem sterilisierenden Mittel gespült wird.

Der Modul kann in ein Gesamtsystem integriert werden, das die Versorgung, Entsorgung und Produktseparation bewerkstelligt und durch eine Steuer-Kontroll-Einrichtung überwacht wird.

Das vorstehend beschriebene Verfahren wird bevorzugt auf Zellen tierischen und humanen Ursprungs (einschließlich genetisch modifizierter Zellen und Hybridoma-Zellen) angewandt, ist jedoch auch geeignet für die Kultivierung pflanzlicher und mikrobiologischer Zellen (Bakterien und Pilze).

Zur Durchführung des erfindungsgemäßen Verfahrens ist eine Vorrichtung geeignet, bei welcher - schlauchförmige Membranen in Modulbauweise im wesentlichen parallel zueinander und im wesentlichen geradlinig angeordnet und deren Enden in Rohrböden gehalten sind, bei der erfindungsgemäß zwischen den schlauchförmigen Membranen ein Trägermaterial für die Zellen angeordnet ist, die Kapillarmembranen und das Trägermaterial für die Zellen mattenförmig angeordnet sind, die Kapillarmembranen die Schußfäden und das Trägermaterial für die Zellen die Kettfäden der Matte sind, die Matte zu einem Membranbündel aufgerollt ist und das Membranbündel in einem Gehäuse angeordnet ist, bei dem alle Trennfugen fluiddicht nach außen mit Polyurethan abgedichtet sind.

Die Erfindung wird anhand der Zeichnung in vereinfachter schematischer Darstellungsweise an einigen Ausführungsformen erläutert; darin zeigt

Figur 1 ein Gelege als Flächengebilde,
Figur 2 ein Gewebe als Flächengebilde,
Figur 3 ein Gewirke als Flächengebilde,
Figur 4 ein aufgewickeltes Flächengebilde,
Figur 5 ein Flächengebilde mit alternierender Anordnung von Membranen und Trägermaterial,
Figur 6 ein Flächengebilde, bei dem die Membranen keinen kreisförmigen Querschnitt aufweisen.

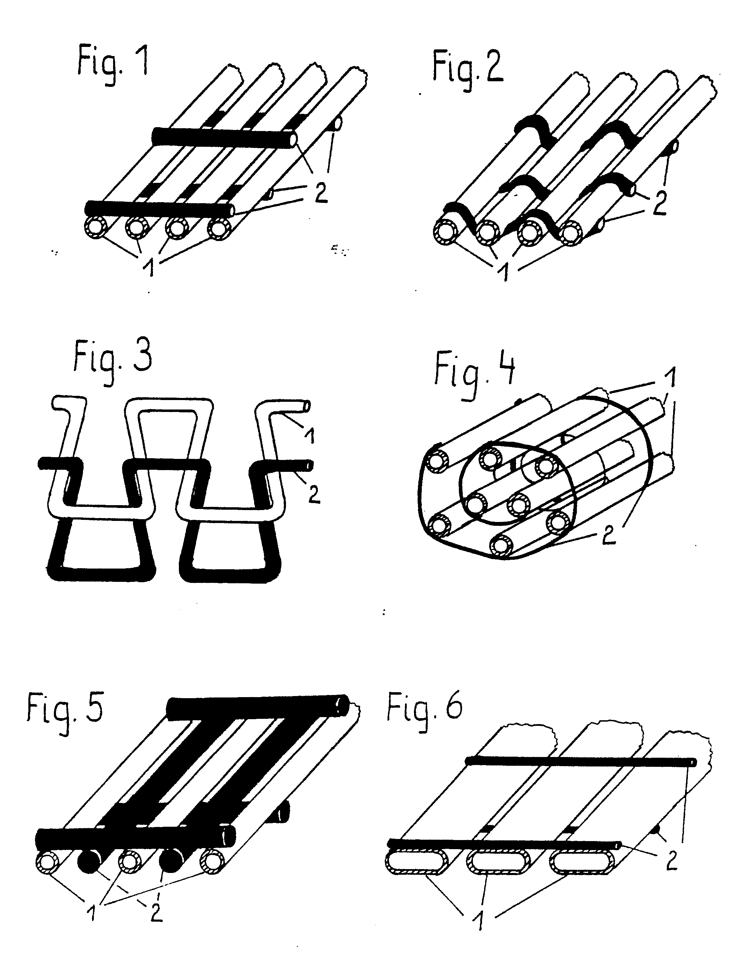

Figur 1 zeigt die Ausführung des Flächengebildes als Gelege mit den schlauchförmigen Membranen 1 und dem Trägermaterial für die Zellen 2.

In Figur 2 ist das Flächengebilde als Gewebe dargestellt, wobei die schlauchförmigen Membranen 1 die Schußfäden und das Trägermaterial für die Zellen 2 die Kettfäden bilden.

Figur 3 zeigt das Flächengebilde in Form eines Gewirkes, bei dem die schlauchförmigen Membranen 1 und das Trägermaterial für die Zellen 2 mit Maschen verbunden sind.

Figur 4 zeigt das Flächengebilde als Matte in aufgewickelter Form, wie es zum Einbau in den Modul verwendet wird. Hierbei zeigt sich, daß bei der bevorzugten Ausführungsform mit den schlauchförmigen Membranen als Schußfäden und dem Trägermaterial als Kettfäden die einfache Herstellung einer Endlosmatte möglich ist, bei der im aufgewickelten Zustand die schlauchförmigen Membranen keinerlei Biegebeanspruchung ausgesetzt sind. Damit eignen sich als Membranmaterial auch solche Stoffe, die keine oder nur geringe Biegekräfte aufnehmen können.

Figure 5 zeigt das Flächengebilde, bei dem sowohl die schlauchförmigen Membranen 1 als auch das Trägermaterial für die Zellen 2 alternierend angeordnet als Schußfäden ausgebildet sind. Damit ist auch die Verwendung von solchen Stoffen als Trägermaterial für die Zellen in Matten möglich, die keine oder nur geringe Biegekräfte aufnehmen können. Die Kettfäden können dann aus einem beliebigen anderen Material bestehen, sofern es grundsätzlich zum Einsatz als Kettfadenmaterial geeignet ist.

Figur 6 zeigt schlauchförmige Membranen 1, die keinen im wesentlichen kreisförmigen Querschnitt aufweisen und mit dem Trägermaterial für die Zellen 2 zu einem Flächengebilde verarbeitet sind. Als Beispiel wurden schlauchförmige Membranen mit einem abgeflachten, runden Querschnitt dargestellt, es sind jedoch ebenso andere beliebige, auch unsymmetrische oder unregelmäßige Querschnittsformen verwendbar.

Die Erfindung wird weiter anhand des nachstehenden Beispiels erläutert.

1. Versuchsdurchführung

Als Kapillarmembran-Modul mit Trägermaterial wurde ein Modul eingesetzt, der mit je ca. 600 CUPROPHAN®-Hohlfäden und Kohlenstoff-Fäden (TENAX®) bestückt war. Die mit diesem Modul erzielten Ergebnisse wurden mit denen eines Kontrollversuchs verglichen, bei dem ein ca. 1 300 CUPROPHAN-Hohlfäden enthaltender, ansonsten übereinstimmender Modul verwendet wurde.

Bei beiden Modulen handelt es sich um zylindrische Labormodule von 14 mm Innendurchmesser und ca. 150 mm wirksamer Länge, die mit geeigneten Anschlüssen am Modulgehäuse versehen sind. Der Füllgrad betrug jeweils etwa 30%. Damit ergab sich ein Extrakapillärvolumen von ca. 16 ml.

Für die Modulherstellung wurden CUPROPHAN-Hohlfäden (für den Kontrollmodul) bzw. CUPROPHAN-Hohlfäden und Kohlenstoff-Fäden in alternierender Anordnung (für den Trägermaterial-Modul) mittels geeigneter Kettfäden zu Matten verbunden, zu Bündeln gerollt und in das Modulgehäuse eingebracht. Die Abdichtung des Extrakapillärraums (EKR) gegen den Intrakapillärraum (IKR) erfolgte durch Verguß mit Polyurethan an den Modulenden.

4

Beide Module wurden für die Kultivierung von L929-Zellen (Maus-Fibroblasten-Zellinie) eingesetzt.

Die Zellen wurden im Extrakapillärraum (EKR) der Module kultiviert. Die Anfangszelldichte betrug jeweils $1 \cdot 10^6$ Zellen/ml. Im EKR wurde als Kulturmedium Minimum Essential Medium Eagle (modifiziert) mit Earle's Salzen (Fa. Flow Laboratories) eingesetzt, dessen Natriumhydrogencarbonat-Konzentration 2.2 g/l betrug und dem nichtessentielle Aminosäuren (1% eines 100-fach-Konzentrats von Fa. Flow Laboratories, Kat.-Nr. 16-810 und 10% fötales Kälberserum zugesetzt werden.

Während der Kultivierung wurden 1.5-1.6 l Medium (Zusammensetzung wie für EKR, jedoch ohne Serumzusatz) mit einer Flußrate von 100 ml/min im Kreislauf durch den Intrakapillärraum (IKR) des Moduls gepumpt. Temperatur, pH-Wert und Sauerstoffgehalt dieses Mediums (IKR-Medium) wurden dabei mit Hilfe des Fermenters BIOSTAT® M mit Gasmischsystem (Hersteller B. Braun Melsungen) auf geeigneten Werten ($37\,°C$, pH 7.2, $pO_2$ 99% Luftsättigung) so weit als möglich konstant gehalten.

Bei beiden Modulläufen wurde das IKR-Medium jeweils an Tag 6, 12 und 20 vollständig gegen frisches Medium ausgetauscht, bei dem Modul mit Trägermaterial entsprechend der stärkeren metabolischen Aktivität (siehe unten) außerdem noch an Tag 16, 23 und 26.

Die Dauer der Kultivierung betrug vier Wochen.

Um das Zellwachstum zu verfolgen, wurden in geeigneten Zeitabständen Proben aus dem IKR-Kreislauf und aus dem EKR gezogen und der Glucose-, Laktat-und Ammoniumgehalt darin bestimmt.

Bei Abbruch der Kultivierung wurden IKR und EKR des Trägermaterial-Moduls leergesaugt und das Modul an beiden Enden an den Vergußstellen aufgesägt, so daß die Matte aus CUPROPHAN-Hohlfäden und Kohlenstoff-Fäden herausgezogen und mikroskopisch untersucht werden konnte.


## 2. Ergebnisse

In Tabelle 1 sind für verschiedene Zeitpunkte während der Kultivierung die bis dahin jeweils insgesamt verbrauchte Menge an Glucose sowie insgesamt produzierte Menge an Laktat bzw. Ammonium angegeben.

Nach 28-tägiger Kultivierung war bei dem Modul mit Trägermaterial verglichen mit dem Kontrollversuch
-die insgesamt verbrauchte Menge an Glucose gut neunmal höher (2315 mg gegenüber 254 mg),
-die insgesamt produzierte Menge an Laktat gut sechsmal größer (1280 mg gegenüber 203 mg) und
-die insgesamt freigesetzte Menge an Ammonium leicht höher (10.9 mmol gegenüber 7.1 mmol).

Die im Kontrollexperiment für Tag 28 erhaltenen Endwerte wurden beim Trägermaterial-Modul schon
-etwa an Tag 9 (Glucose),
-zwischen Tag 15 und 2o (Laktat) bzw.
-an Tag 20 (Ammonium)
erreicht.

Im Zeitraum von Tag 26 bis Tag 28 lag im Trägermaterial-Modul verglichen mit dem Kontrollversuch
-die Glucoseverbrauchsrate gut fünfzehnmal höher (213 mg/Tag gegenüber 14 mg/Tag),
-die Laktatproduktionsrate gut achtmal höher (137 mg/Tag gegenüber 16 mg/Tag und
-die Ammoniumproduktionsrate gut dreimal höher (0.54 mmol/Tag gegenüber 0.16 mmol/Tag).

Insgesamt wurde also in dem Modul mit Trägermaterial eine - verglichen mit dem Kontrollversuch - erheblich höhere Stoffwechselgesamtaktivität erzielt, was auf eine deutlich größere Zelldichte schließen läßt.

Bei der lichtmikroskopischen Beurteilung der aus dem Trägermaterial-Modul entnommenen Matte wurde festgestellt, daß der überwiegende Teil (schätzungsweise mehr als 90%) der Zellen an den Kohlenstoff-Fäden haftete, während die CUPROPHAN-Hohlfäden weitgehend zellfrei waren.

CUPROPHAN und TENAX sind eingetragene Warenzeichen der Enka AG.

## Tabelle 1

| Tag | Gesamtmenge an verbrauchter Glucose (mg) | | Gesamtmenge an produziertem Laktat (mg) | | Gesamtmenge an produziertem Ammonium (mmol) | |
|---|---|---|---|---|---|---|
| | Modul 1 | Modul 2 | Modul 1 | Modul 2 | Modul 1 | Modul 2 |
| 9 | 71 | 234 | 68 | 30 | 2.10 | 2.32 |
| 15 | 92 | 481 | 86 | 116 | 3.65 | 4.78 |
| 20 | 104 | 926 | 105 | 410 | 5.12 | 7.10 |
| 23 | 191 | 1352 | 138 | 691 | 6.13 | 8.28 |
| 26 | 227 | 1890 | 171 | 1006 | 6.77 | 9.79 |
| 28 | 254 | 2315 | 203 | 1280 | 7.08 | 10.86 |

Modul 1: Kapillarmembran-Modul ohne Trägermaterial
(bestückt mit CUPROPHAN-C1-Hohlfäden)

Modul 2: Kapillarmembran-Modul mit Trägermaterial
(bestückt mit CUPROPAN-C1-Hohlfäden und Kohlenstoff-Fäden)

**Ansprüche**

1. Verfahren zum Kultivieren von tierischen Zellen zur Vermehrung derselben und/oder Gewinnung von gewünschten Wertstoffen aus den Zellen, wobei sich in einem wenigstens eine schlauchförmige Membran enthaltenden Modul Zellen im Raum außerhalb der wenigstens einen schlauchförmigen Membran in einem wäßrigen Kulturmedium befinden und durch das Innere der wenigstens einen schlauchförmigen Membran die Versorgung der Zellen mit Nährstoffen und die Entsorgung von Metaboliten erfolgt, dadurch gekennzeichnet, daß ein Modul verwendet wird, bei dem im Raum außerhalb der wenigstens einen - schlauchförmigen Membran ein Trägermagerial für die Zellen angeordnet ist, auf dem die Zellen bevorzugt adhärieren.

2. Verfahren nach Anspruch 1, dadurch gekennzeichnet, daß die schlauchförmige Membran/schlauchförmigen Membranen als Kapillarmembran/Kapillarmembranen ausgebildet ist/sind.

3. Verfahren nach Anspruch 1, dadurch gekennzeichnet, daß das Trägermaterial für die Zellen aus verseiftem Celluloseacetat besteht.

4. Verfahren nach Anspruch 1, dadurch gekennzeichnet, daß das Trägermaterial für die Zellen aus einem fadenbildenden gesättigten Polyester und/oder Copolyester besteht.

5. Verfahren nach Anspruch 1, dadurch gekennzeichnet, daß das Trägermaterial für die Zellen aus einem fadenbildenden Polyurethan besteht.

6. Verfahren nach Anspruch 1, dadurch gekennzeichnet, daß das Trägermaterial für die Zellen aus einem fadenbildenden Polyacrylnitril besteht.

7. Verfahren nach Anspruch 1, dadurch gekennzeichnet, daß das Trägermaterial für die Zellen aus einem aromatischen Polyamid besteht.

8. Verfahren nach Anspruch 1, dadurch gekennzeichnet, daß das Trägermaterial für die Zellen aus Kieselsäure besteht.

9. Verfahren nach Anspruch 1, dadurch gekennzeichnet, daß das Trägermaterial für die Zellen aus Kohlenstoff besteht.

10. Verfahren nach einem oder mehreren der Ansprüche 3 bis 9, dadurch gekennzeichnet, daß das Trägermaterial für die Zellen aus Fasern und/oder Filamenten aufgebaut ist.

11. Verfahren nach Anspruch 10, dadurch gekennzeichnet, daß die Fasern und/oder Filamente in Fadenform eingesetzt werden.

12. Verfahren nach einem oder mehreren der Ansprüche 3 bis 11, dadurch gekennzeichnet, daß das Trägermaterial für die Zellen aus beschichteten Textilfäden besteht.

13. Verfahren nach Anspruch 12, dadurch gekennzeichnet, daß das Trägermaterial für die Zellen aus polyurethanbeschichteten Textilfäden besteht.

14. Verfahren nach Anspruch 12, dadurch gekennzeichnet, daß das Trägermaterial für die Zellen aus mit biologischen Substanzen beschichteten Textilfäden besteht.

15. Verfahren nach einem oder mehreren der Ansprüche 3 bis 14, dadurch gekennzeichnet, daß das Trägermaterial fär die Zellen mit unterschiedlichen Fadentypen in alternierender Anordnung ausgebildet ist.

16. Verfahren nach einem oder mehreren der Ansprüche 1 bis 2, dadurch gekennzeichnet, daß die - schlauchförmige Membran/schlauchförmigen Membranen aus regenerierter Cellulose besteht/bestehen.

17. Verfahren nach einem oder mehreren der Ansprüche 1 bis 2, dadurch gekennzeichnet, daß die - schlauchförmige Membran/schlauchförmigen Membranen aus mikroporösen synthetischen Polymeren besteht/bestehen.

18. Verfahren nach Anspruch 17, dadurch gekennzeichnet, daß der maximale Porendurchmesser 0,05 bis 0,2 μm beträgt.

19. Verfahren nach einem oder mehreren der Ansprüche 1 bis 2, dadurch gekennzeichnet, daß unterschiedliche Membrantypen für die Versorgung und Entsorgung verwendet werden.

20. Verfahren nach einem oder mehreren der Ansprüche 1 bis 19, dadurch gekennzeichnet, daß die - schlauchförmige Membran/schlauchförmigen Membranen und das Trägermaterial für die Zellen zusammen wenigstens ein Flächengebilde bilden.

21. Verfahren nach Anspruch 20, dadurch gekennzeichnet, daß das Flächengebilde als Gelege, Gewebe, Gewirke oder Raschelware ausgebildet ist.

22. Verfahren nach Anspruch 21, dadurch gekennzeichnet, daß die schlauchförmigen Membranen die Schußfäden und das Trägermaterial für die Zellen die Kettfäden bilden.

23. Verfahren nach Anspruch 21, dadurch gekennzeichnet, daß die schlauchförmigen Membranen und die das Trägermaterial für die Zellen bildenden Fäden in zueinander im wesentlichen paralleler und gegebenenfalls periodischer Anordnung als Schußfäden angeordnet sind.

24. Verfahren nach Anspruch 21, dadurch gekennzeichnet, daß die schlauchförmigen Membranen und ein erster Teil der das Trägermaterial für die Zellen bildenden Fäden in zueinander im wesentlichen paralleler und gegebenenfalls periodischer Anordnung als Schußfäden sowie ein zweiter Teil der das Trägermaterial für die Zellen bildenden Fäden als Kettfäden angeordnet sind.

25. Verfahren nach einem der Ansprüche 22 bis 24, dadurch gekennzeichnet, daß sich das Verhältnis des Durchmessers der das Trägermaterial für die Zellen bildenden Fäden zum Durchmesser der - schlauchförmigen Membranen vorzugsweise im Bereich zwischen 0,4 und 2,0 liegt.

26. Verfahren nach einem der Ansprüche 22 bis 25, dadurch gekennzeichnet, daß die Abstände der Schußfäden untereinander vorzugsweise das 0,5-bis 3-fache des äußeren Membrandurchmessers betragen.

27. Verfahren nach einem oder mehreren der Ansprüche 22 bis 26, dadurch gekennzeichnet, daß die Abstände der Kettfäden untereinander vorzugsweise mindestens das 1,0-fache und höchstens das 100-fache des Kettfadendurchmessers betragen.

28. Verfahren nach einem oder mehreren der Ansprüche 1 bis 27, dadurch gekennzeichnet, daß die - schlauchförmigen Membranen durch Abstandshalter in definierten gegenseitigen Abständen festgelegt sind.

29. Verfahren nach Anspruch 28, dadurch gekennzeichnet, daß die Abstandshalter ganz oder teilweise das Trägermaterial für die Zellen bilden.

30. Verfahren nach einem oder mehreren der Ansprüche 20 bis 29, dadurch gekennzeichnet, daß zwischen den Flächengebildelagen Abstandshalter angeordnet sind.

31. Verfahren nach einem oder mehreren der Ansprüche 1 bis 30, dadurch gekennzeichnet, daß Zellen an das Trägermaterial für die Zellen bevorzugt adhärieren, insbesondere in und nach der Inokulationsphase.

32. Verfahren nach einem oder mehreren der Ansprüche 1 bis 31, dadurch gekennzeichnet, daß die Versorgung und/oder Entsorgung mittels einer Steuer-Kontroll-Einheit überwacht wird.

7

33. Vorrichtung, geeignet zum Durchführen des Verfahrens nach einem oder mehreren der Ansprüche 1 bis 32, bei welcher schlauchförmige Membranen in Modulbauweise im wesentlichen parallel zueinander und im wesentlichen geradlinig verlaufend angeordnet und deren Enden in Rohrböden gehalten sind, dadurch gekennzeichnet, daß zwischen den schlauchförmigen Membranen ein Trägermaterial für die Zellen angeordnet ist.

34. Vorrichtung nach Anspruch 33, dadurch gekennzeichnet, daß die schlauchförmigen Membranen und das Trägermaterial für die Zellen mattenförmig angeordnet sind und daß die schlauchförmigen Membranen die Schußfäden und das Trägermaterial für die Zellen die Kettfäden der Matte sind.

35. Vorrichtung nach Anspruch 34, dadurch gekennzeichnet, daß die Matte zu einem Membranbündel aufgerollt ist.

36. Vorrichtung nach Anspruch 35, dadurch gekennzeichnet, daß das Membranbündel in einem Gehäuse angeordnet ist, bei dem alle Trennfugen fluiddicht nach außen mit Polyurethan abgedichtet sind.

Fig. 1

Fig. 2

Fig. 3

Fig. 4

Fig. 5

Fig. 6